(19) Europäisches Patentamt
European Patent Office
Office européen des brevets

(11) Publication number: 0 047 358
B1

(12) # EUROPEAN PATENT SPECIFICATION

(45) Date of publication of patent specification: 19.09.84

(21) Application number: 81104024.5

(22) Date of filing: 26.05.81

(51) Int. Cl.$^3$: C 07 D 401/12, A 61 K 31/44 // (C07D401/12, 209/28, 213/32),(C07D401/12, 213/61, 209/28)

(54) **Indol acetic derivatives, process for producing the same and pharmaceutical compositions comprising the same.**

(30) Priority: 10.09.80 DE 3034005

(43) Date of publication of application: 17.03.82 Bulletin 82/11

(45) Publication of the grant of the patent: 19.09.84 Bulletin 84/38

(84) Designated Contracting States: AT BE CH FR GB IT LI LU NL SE

(56) References cited:
EP-A-0 000 741
US-A-3 966 956

(73) Proprietor: A. Nattermann & Cie. GmbH
Nattermannallee 1
D-5000 Köln 30 (DE)

(72) Inventor: Betzing, Hans, Dr.
Heidestock 7
D-5014 Kerpen-Horrem (DE)
Inventor: Leyck, Sigurd, Dr.
Am Quechenhauf 21
D-5024 Brauweiler (DE)

(74) Representative: Redies, Bernd, Dr. rer. nat. et al
Redies, Redies, Türk & Gille Brucknerstrasse 20
D-4000 Düsseldorf 13 (DE)

EP 0 047 358 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention is related to new indol acetic acid derivatives having the general formula I

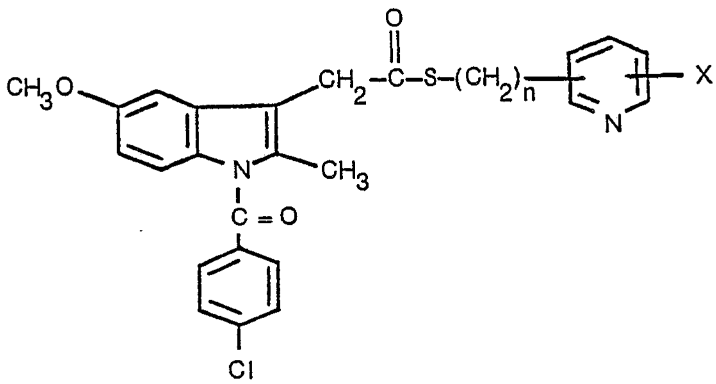

I

wherein

X is H, F, Cl or a $C_{1-3}$ alkyl group, preferably H, Cl or $CH_3$,

n is an integer from 0 to 3,

and the pharmaceutically compatible, i.e. pharmacologically acceptable salts thereof such as the hydrochloride, fumarate, tartrate, succinate, 2-ketoglutarate, citrate, salicylate or acetylsalicylate.

Most preferred in view of their valuable properties are those compounds of formula I wherein X is hydrogen and n is 1 and the pharmacologically acceptable salts thereof.

The invention is further related to a process for producing the compounds of formula I comprising either

a) to subject 1-(p-chlorobenzoyl)-5-methoxy-2-methyl-indol-3-acetic acid chloride to reaction with an alkali salt of a compound of the general formula II

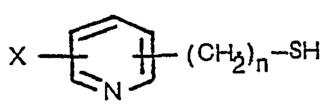

II

wherein X and n have the same meaning as in formula I, in particular the sodium or potassium salt thereof, in an organic inert solvent such as a cyclic or aliphatic hydrocarbon or halogenated hydrocarbon, in particular toluene, benzene, hexane, chloroform, dimethylformamide or dichloromethane, or

b) to subject 1-(p-chlorobenzoyl)-5-methoxy-2-methyl-indol-3-acetic acid to reaction with a compound of the above general formula II in the presence of a carbodiimide, in particular N,N'-dicyclohexyl carbodiimide, or in the presence of N,N-dimethylphosphoramide dichloride and a weak base (such as pyridine, triethylamine or potassium carbonate) in an organic inert solvent such as toluene, benzene, hexane, dimethylformamide or dichloromethane, or

c) to subject an 1-(p-chlorobenzoyl)-5-methoxy-2-methyl-indol-3-acetic acid $C_{1-4}$-alkylester, in particular the methyl or ethyl ester, to reaction with a compound of the above general formula II in an organic inert solvent such as toluene, benzene, hexane or dimethylformamide, in particular dichloromethane, in the presence of trimethyl aluminium dissolved in a hydrocarbon, and converting the resulting product, if desired, to pharmacologically acceptable salts.

The above embodiments of the process according to the present invention are carried out at a temperature ranging from 0°C. to 60°C., preferably from 20°C. to 25°C. The process embodiment c) preferably is carried out in the presence of a protective gas.

The compounds according to the present invention have an antithrombotic, antiarterisoclerotic, analgetic and in particular antiphlogistic activity. They are in particular useful in the treatment of rheumatic diseases such as arthrosis or chronic polyarthritis. The new compounds are in particular characterized by a very good compatability since they show a low toxicity and, contrary to known antiphlogistic drugs, no incompatibility to the stomach, i.e. they for instance do not produce ulcera of the stomach or gastrointestinal irritations.

The compounds according to the present invention can be converted into pharmaceutical preparations containing the same in manners known per se. Thus, the active compounds according to the present invention may be used as such or in combination with suitable pharmaceutical diluents and/or carrier materials and may be formulated in usual manners. The compounds according to the present invention may be used both in human veterinary medicine in any desired form such as in systemic form provided that the formulation and maintenance of a sufficient blood and tissue level is produced. This is possible by oral, rectal or parenteral administration of suitable dosages. It is preferred to use

pharmaceutical preparations allowing the administration of single dosages in suitable forms of administration such as tablets, dragées, capsules, suppositories, granulates, solutions, emulsions, suspensions, sols or gels. The dosage of adminsitration in general is between 20 and 500 mg. per day, preferably between 30 and 200 mg. per day and may be administered in a single dose or several doses, preferably in two or three daily doses.

Suitable carrier materials for the preparation of orally administratable preparations, for instance tablets, capsules, granulates or powders, are for instance calcium carbonate, calcium phosphate, starch, sugar, lactose, talcum, magnesium stearate, gelatine, polyvinylpyrrolidone, gum-arabic, sorbitol, microcrystalline cellulose, polyethylene glycol, carboxymethylcellulose and shellac. Tablets may be coated in usual manners. Liquid products for oral administrations may be aqueous or oily suspensions or solutions. They may also be powderous products with a filler material obtained by deep freeze drying which products are dissolved before administration.

The pharmaceutical preparations according to the present invention may also be suppositories for rectal administration containing pharmaceutically acceptable carrier materials such as polyethyleneglycol, lanolin, coconut butter or witepsol. The products may also be prepared for external administration in the form of ointments or creams which are produced in usual manners with usual additives.

The following examples serve to further illustrate the present invention without however limiting the same thereto.

Example 1

Production of 1-(p-chlorobenzoyl)-5-methoxy-2-methyl-3-indol acetic acid-2-pyridylmethylthioester according to process embodiment b).

6.5 g. (0.018 mol.) of 1-(p-chlorobenzoyl)-5-methoxy-2-methyl-3-indol-acetic acid are dissolved in 60 ml. of chloroform. After the addition of 2.9 g. (0.023 mol.) of 2-mercaptomethylpyridine and 4.9 g. (0.023 mol.) of N,N'-dicyclohexyl carbodiimide, the reaction mixture is stirred for 24 hours with the exclusion of moisture. The precipitated material is filtered off and the solvent of the resulting solution is distilled in a vacuum. The resulting residue is subjected to chromatography over a silicic acid gel column using a 1:1-mixture of chloroform and hexane as eluant.

Yield: 7.1 g. (84.7% of the theoretical)

F.p.: 153 to 154°C.

Elementary analysis: $C_{25}H_{22}O_3N_2SCl$

| | | | | |
|---|---|---|---|---|
| Calculated: | C 63.21%, | H 4.57%, | N 5.88%, | S 6.72% |
| Found: | C 64.44%, | H 4.74%, | N 6.01%, | S 6.88% |

Example 2

Preparation of 1-(p-chlorobenzoyl)-5-methoxy-2-methyl-3-indol acetic acid-3-pyridylmethylthioester according to process embodiment a).

34.2 g (0.1 mol.) of 1-(p-chlorobenzoyl)-5-methoxy-2-methyl-3-indol acetic acid chloride (produced from the corresponding indol acetic acid derivative by reaction with oxalylchloride) are dissolved in 500 ml. of anhydrous dichloromethane, 16.2 g. (0.12 mol.) of the sodium salt of 3-mercaptomethylpyridine are added thereto with the exclusion of moisture and the reaction mixture is stirred for 6 hours at room temperature with the exclusion of moisture. The precipitated sodium chloride is filtered off and the solvent of the resulting solution is distilled off in a vacuum. The resulting reaction product is purified by means of chromatography on silicic acid gel as adsorbing agent and chloroform as eluant.

Yield: 33 g. (70.8% of the theoretical)

F.p.: 127 to 128°C.

Hydrochloride: F.p. 159 to 161°C. (from methanol/ether).

Example 3

Production of 1-(p-chlorobenzoyl)-5-methoxy-2-methyl-3-indol acetic acid-3-(3-pyridyl)propylthioester by process embodiment c).

5.76 g. (0.02 mol.) of trimethylaluminum (corresponding to 8 ml. of a 25% solution of trimethylaluminum in hexane) are dissolved in 40 ml. of anhydrous methylenechloride and cooled to 0°C. 2.74 g. (0.02 mol.) of 3-(3-pyridyl)-1-propylmercaptane are added thereto with stirring, exclusion of moisture and in a nitrogen gas atmosphere. The reaction mixture is heated to room temperature within 15 to 20 minutes whereafter a solution of 8.12 g (0.02 mol.) of 1-(p-chlorobenzoyl)-5-methoxy-2-methyl-1-indol acetic acid ethylester dissolved in 5 ml. of methylene chloride is added thereto. The reaction mixture is stirred, at the same time introducing nitrogen gas into the reaction vessel as protective gas, stirring being continued until the reaction is finished as controlled by thin layer chromatography. About 100 ml. of ether are added and the resulting solution is first shaken with a 3% aqueous hydrochloride acid and then with a 5% aqueous lye.

The ethereal phase is dried over anhydrous sodium sulfate and the solvent is distilled off. The remaining residue is purified by chromatography using a column containing silicic acid gel as adsorption agent and chloroform as eluant.

Yield: 8.5 g. (88.9% of the theoretical) of a slightly yellow hydroscopic product having the RF-value 0,813 (using the prefabricated silicic acid gel plates), eluant: chloroform/methanol 95:5
molecular analysis: $C_{27}H_{26}O_3N_2S_1Cl_1$
Calculated: C 65.65%, H 5.31%, N 5.6%, S 6.48%
Found: C 63.91%, H 5.18%, N 5.42%, S 6.52%
The salicylate melts at F.p.: 224 to 225°C.
In accordance with examples 1 to 3 the following compounds have been prepared:

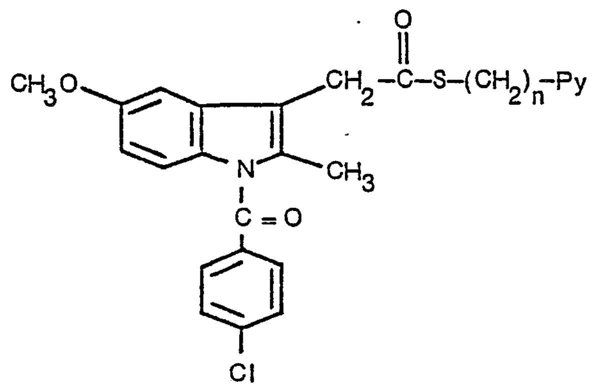

| Example | n | Py | Salt | F.p. (°C.) |
|---|---|---|---|---|
| 4 | 2 | 2-pyridyl | hydrochloride | 125—126[+)] |
| 5 | 1 | 4-pyridyl | — | 207—209 |
| 6 | 1 | 6-methyl-3-pyridyl | — | 136—138 |
| 7 | 0 | 2-pyridyl | — | 175—176 |
| 8 | 1 | 6-chloro-2-pyridyl | — | 168—170 |
| 9 | 1 | 5-fluoro-2-pyridyl | — | 165—166 |
| 10 | 1 | 3-pyridyl | fumarate | 127 |
| 11 | 2 | 2-pyridyl | acetylsalicilate | 210—211 |

[+)]hydroscopic

Example 12

Tablets

| | |
|---|---|
| 1-(p-Chlorobenzoyl)-5-methoxy-2-methyl-3-indol acetic acid-3-pyridylmethylthioester | 30 mg. |
| lactose | 150 mg. |
| crystalline cellulose | 50 mg. |
| calcium carboxymethylcellulose | 7 mg. |
| magnesium stearate | 3 mg. |

The above components are mixed in usual manners, granulated and filled into hard gelatine capsules.

Example 13

Capsules

| | |
|---|---|
| 1-(p-Chlorobenzoyl)-5-methoxy-2-methyl-3-indol acetic acid-3-pyridylmethylthioester | 50 mg. |
| talcum | 5 mg. |
| Aerosil 200 | 10 mg. |

The above components are mixed, granulated and filled into hard gelatine capsules.

**Claims for the Contracting States: BE CH FR GB IT LI LU NL SE**

1. Indol acetic acid derivative having the general formula I

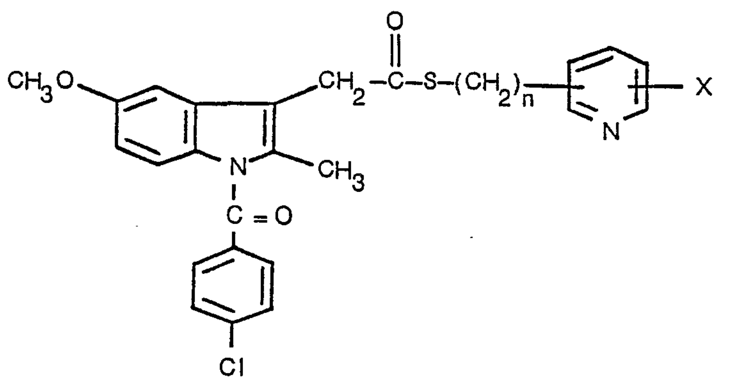

I

wherein
X is H, F, Cl or a $C_{1-3}$-alkyl group
n is an integer from 0 to 3,
and the pharmacologically acceptable salts thereof.

2. 1-(p-Chlorobenzoyl)-5-methoxy-2-methyl-3-indol acetic acid-2-pyridylmethylthioester and the pharmacologically acceptable salts thereof.

3. 1-(p-Chlorobenzoyl)-5-methoxy-2-methyl-3-indol acetic acid-3-pyridylmethylthioester and the pharmacologically acceptable salts thereof.

4. 1-(p-Chlorobenzoyl)-5-methoxy-2-methyl-3-indol acetic acid-2-(3-pyridyl)-ethylthioester and the pharmacologically acceptable salts thereof.

5. 1-(p-Chlorobenzoyl)-5-methoxy-2-methyl-3-indol acetic acid-3-(3-pyridyl)-propylthioester and the pharmacologically acceptable salts thereof.

6. 1-(p-Chlorobenzoyl)-5-methoxy-2-methyl-3-indol acetic acid-4-pyridylmethylthioester and the pharmacologically acceptable salts thereof.

7. 1-(p-Chlorobenzoyl)-5-methoxy-2-methyl-3-indol acetic acid-(6-methyl-3-pyridyl)-methylthioester and the pharmacologically acceptable salts thereof.

8. 1-(p-Chlorobenzoyl)-5-methoxy-2-methyl-3-indol acetic acid-2-pyridylthioester and the pharmacologically acceptable salts thereof.

9. 1-(p-Chlorobenzoyl)-5-methoxy-2-methyl-3-indol acetic-(6-chloro-2-pyridyl)-methylthioester and the pharmacologically acceptable salts thereof.

10. Process for producing a compound according to any of claims 1 to 9 comprising

a) subjecting 1-(p-chlorobenzoyl)-5-methoxy-2-methyl indol-3-acetic acid chloride to reaction with an alkali salt of a compound of the general formula II

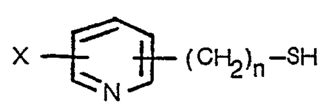

II

wherein X and n have the same meaning as in claim 1, in an organic inert solvent or

b) subjecting 1-(p-chlorobenzoyl)-5-methoxy-2-methyl indol-3-acetic acid to reaction with a compound of the above general formula II in the presence of a carbodiimide, or in the presence of N,N-dimethylphosphoramidedichloride together with a weak base, in an organic inert solvent or

c) subjecting an 1-(p-chlorobenzoyl)-5-methoxy-2-methyl indol-3-acetic acid-$C_{1-4}$-alkylester to reaction with a compound of the above formula II in the presence of a trimethylaluminum in an inert organic solvent.

11. Pharmaceutical compositions comprising at least one compound according to any of claims 1 to 9 together with pharmaceutically acceptable diluant and/or carrier materials.

**Claim for the Contracting State: AT**

Process for producing an indol acetic acid derivative having the general formula I

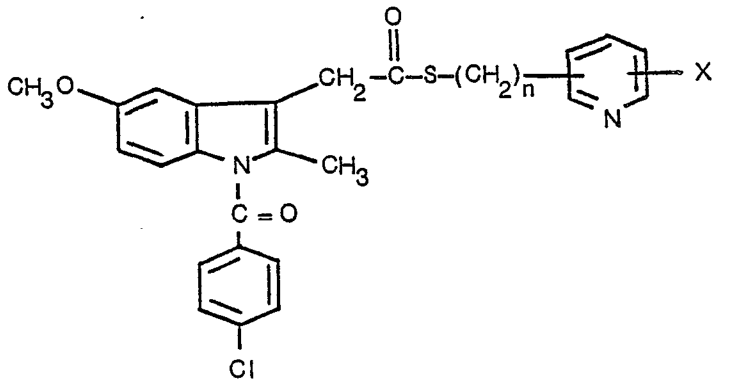

I

wherein

X is H, F, Cl or a $C_{1-3}$-alkyl group

n is an integer from 0 to 3

and the pharmacologically acceptable salts thereof.

Comprising

a) subjecting 1-(p-chlorobenzoyl)-5-methoxy-2-methyl indol-3-acetic acid chloride to reaction with an alkali salt of a compound of the general formula II

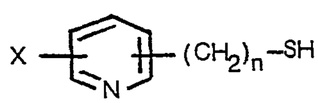

II

wherein X and n have the same meaning as above in an organic inert solvent or

b) subjecting 1-(p-chlorobenzoyl)-5-methoxy-2-methyl indol-3-acetic acid to reaction with a compound of the above general formula II in the presence of a carbodiimide, or in the presence of N,N-dimethylphosphoramidedichloride together with a weak base, in an organic inert solvent or

c) subjecting an 1-(p-chlorobenzoyl)-5-methoxy-2-methyl indol-3-acetic acid-$C_{1-4}$-alkylester to reaction with a compound of the above formula II in the presence of trimethylaluminum in an inert organic solvent.

**Patentansprüche für die Vertragsstaaten: BE CH FR GB IT LI LU NL SE**

1. Indolessigsäure-Derivat mit der allgemeinen Formel

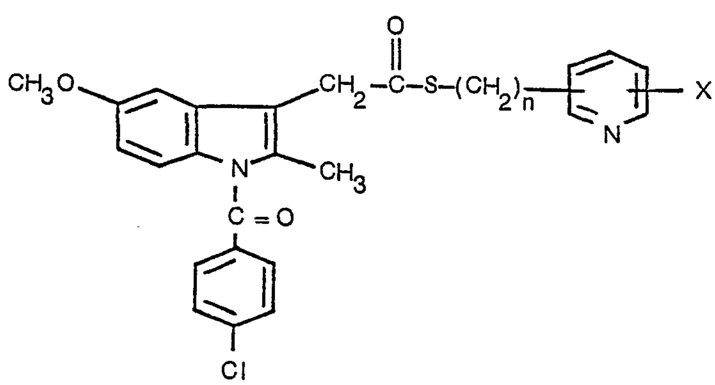

I

worin bedeuten:

X, H, F, Cl oder eine $C_{1-3}$-Alkylgruppe,

n eine ganze Zahl von 0 bis 3,

sowie die pharmakologisch akzeptablen Salze desselben.

2. 1-(p-Chlorobenzoyl)-5-methoxy-2-methyl-3-indolessigsäure-2-pyridylmethylthioester und die pharmakologisch akzeptablen Salze desselben.

3. 1-(p-Chlorobenzoyl)-5-methoxy-2-methyl-3-indolessigsäure-3-pyridylmethylthioester und die pharmakologisch akzeptablen Salze desselben.

4. 1-(p-Chlorobenzoyl)-5-methoxy-2-methyl-3-indolessigsäure-2-(3-pyridyl)ethylthioester und die pharmakologisch akzeptablen Salze desselben.

5. 1-(p-Chlorobenzoyl)-5-methoxy-2-methyl-3-indolessigsäure-3-(3-Pyridyl)propylthioester und die pharmakologisch akzeptablen Salze desselben.

6. 1-(p-Chlorobenzoyl)-5-methoxy-2-methyl-3-indolessigsäure-4-pyridylmethylthioester und die pharmakologisch akzeptablen Salze desselben.

7. 1-(p-Chlorobenzoyl)-5-methoxy-2-methyl-3-indolessigsäure-(6-methyl-3-pyridyl)-methylthioester und die pharmakologisch akzeptablen Salze desselben.

8. 1-(p-Chlorobenzoyl)-5-methoxy-2-methyl-3-indolessigsäure-2-pyridylthioester und die pharmakologisch akzeptablen Salze desselben.

9. 1-(p-Chlorobenzoyl)-5-methoxy-2-methyl-3-indolessigsäure-(6-chloro-2-pyridyl)methylthioester und die pharmakologisch akzeptablen Salze desselben.

10. Verfahren zur Herstellung einer Verbindung nach einen der Ansprüche 1 bis 9, das umfaßt

a) die Umsetzung von 1-(p-Chlorobenzoyl)-5-methoxy-2-methylindol-3-essigsäurechlorid mit einem Alkalisalz einer Verbindung der allgemeinen Formel II

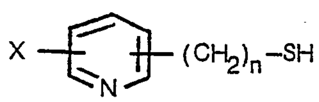

II

worin X und n die gleichen Bedeutungen wie in Anspruch 1 haben, in einem organischen inerten Lösungsmittel oder

b) die Umsetzung von 1-(p-Chlorobenzoyl)-5-methoxy-2-methylindol-3-essigsäure mit einer Verbindung der oben angegebenen allgemeinen Formel II in Gegenwart eines Carbodiimids oder in Gegenwart von N,N-Dimethylphosphoramiddichlorid zusammen mit einer schwachen Base in einem organischen inerten Lösungsmittel oder

c) die Umsetzung eines 1-(p-Chlorobenzoyl)-5-methoxy-2-methylindol-3-essigsäure-$C_{1-4}$-alkylesters mit einer Verbindung der obigen Formel II in Gegenwart von Trimethylaluminium in einem inerten organischen Lösungsmittel.

11. Pharmazeutische Mittel, die mindestens eine Verbindung nach einem der Ansprüche 1 bis 9 zusammen mit pharmazeutisch akzeptablen Verdünnungsmitteln und/oder Trägermaterialien enthalten.

**Patentanspruch für den Vertragsstaat: AT**

Verfahren zur Herstellung eines Indolesseigsäurederivats der allgemeinen Formel I

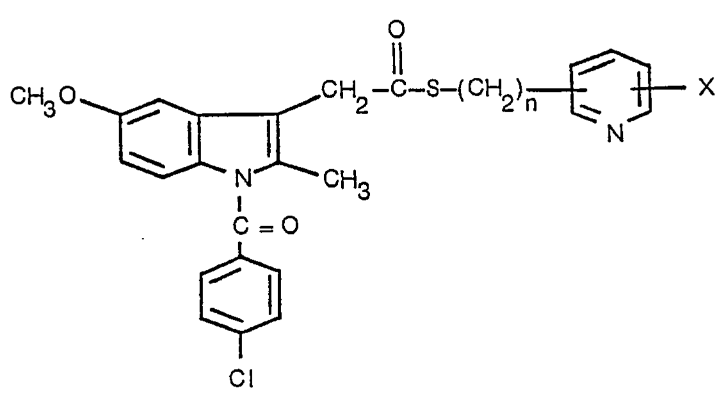

I

worin bedeuten:

X, H, F, Cl oder eine $C_{1-3}$-Alkylgruppe,

n eine ganze Zahl von 0 bis 3

sowie die pharmakologisch verträglichen Salze davon, das umfaßt

a) die Umsetzung von 1-(p-Chlorobenzoyl)-5-methoxy-2-methylindol-3-essigsäurechlorid mit einem Alkalisalz einer Verbindung der allgemeinen Formel II

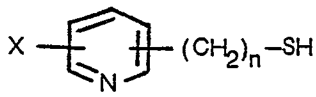

II

worin X und n die gleichen Bedeutungen wie in der Formel I haben, in einem organischen inerten Lösungsmittel oder

b) die Umsetzung von 1-(p-Chlorobenzoyl)-5-methoxy-2-methylindol-3-essigsäure mit einer Verbindung der oben angegebenen allgemeinen Formel II in Gegenwart eines Carbodiimids oder in Gegenwart von N,N-Dimethylphosphoramidodichlorid zusammen mit einer schwachen Base in einem organischen inerten Lösungsmittel oder

c) die Umsetzung eines 1-(p-Chlorobenzoyl)-5-methoxy-2-methylindol-3-essigsäure-$C_{1-4}$-alkylesters mit einer Verbindung der oben angegebenen Formel II in Gegenwart von Trimethylaluminium in einem inerten organischen Lösungsmittel.

**Revendications pour les Etats contractants: BE CH FR GB IT LI LU NL SE**

1. Dérivé d'acide acétique d'indole possédant la formule générale I

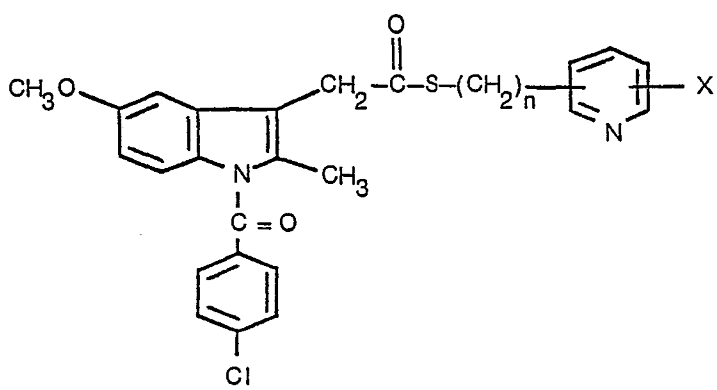

I

dans laquelle

X est H, F, Cl ou un groupe alkyle en $C_{1-3}$

n est un nombre entier de 0 à 3,

et leurs sels acceptables d'un point de vue pharmacologique.

2. 2-pyridylméthylthioester d'acide acétique de 1-(p-chlolrobenzoyl)-5-méthoxy-2-méthyl-3-indole et ses sels acceptables d'un point de vue pharmacologique.

3. 3-pyridylméthylthioester d'acide acétique de 1-(p-chlorobenzoyl)-5-méthoxy-2-méthyl-3-indole et ses sels acceptables d'un point de vue pharmacologique.

4. 2-(3-pyridyl)-éthylthioester d'acide acétique de 1-(p-chlorobenzoyl)-5-méthoxy-2-méthyl-3-indole et ses sels acceptables d'un point de vue pharmacologique.

5. 3-(3-pyridyl)-propylthioester d'acide acétique de 1-(p-chlorobenzoyl)-5-méthoxy-2méthyl-3-indole et ses sels acceptables d'un point de vue pharmacologique.

6. 4-pyridylméthylthioester d'acide acétique de 1-(p-chlorobenzoyl)-5-méthoxy-2-méthyl-3-indole et ses sels acceptables d'un point de vue pharmacologique.

7. (6-méthyl-3-pyridyl)-méthylthioester d'acide acétique de 1-(p-chlorobenzoyl)-5-méthoxy-2-méthyl-3-indole et ses sels acceptables d'un point de vue pharmacologique.

8. 2-pyridylthioester d'acide acétique de 1-(p-chlorobenzoyl)-5-méthoxy-2-méthyl-3-indole et ses sels acceptables d'un point de vue pharmacologique.

9. (6-chloro-2-pyridyl)-méthylthioester d'acide acétique de 1-(p-chlorobenzoyl)-5-méthoxy-2-méthyl-3-indole et ses sels acceptables d'un point de vue pharmacologique.

10. Procédé de production d'un composé selon l'une des revendications 1 à 9, caractérisé en ce qu'il comprend

a) la soumission de chlorure d'acide 1-(p-chlorobenzoyl)-5-méthoxy-2-méthyl-indol-3-acétique à une réaction avec un sel alcalin d'un composé de formule générale II

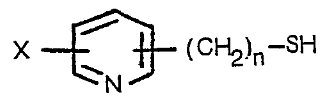

II

dans laquelle X et n ont la même signification que dans la revendication 1, dans un solvant inerte organique ou

b) la soumission d'un acide 1-(p-chlorobenzoyl)-5-méthoxy-2-méthyl-indol-3-acétique à une réaction avec un composé de la formule générale II ci-dessus en présence d'un carbodiimide, ou en présence de N,N-diméthylphosphoramidedichlorure en même temps qu'une base faible, dans un solvant organique inerte ou

c) la soumission d'un ester d'alkyle en $C_{1-4}$ d'acide 1-(p-chlorobenzoyl)-5-méthoxy-2-méthyl-indol-3-acétique à une réaction avec un composé de la formule II ci-dessus en présence de triméthyl-aluminium dans un solvant organique inerte.

11. Compositions pharmaceutiques caractérisées en ce qu'elles comprennent au moins un composé selon l'une des revendications 1 à 9 ainsi que des matériaux diluants et/ou véhicules acceptables d'un point de vue pharmaceutique.

**Revendication pour l'Etat contractant: AT**

1. Procédé de préparation d'un dérivé d'acide acétique d'indole de formule générale I

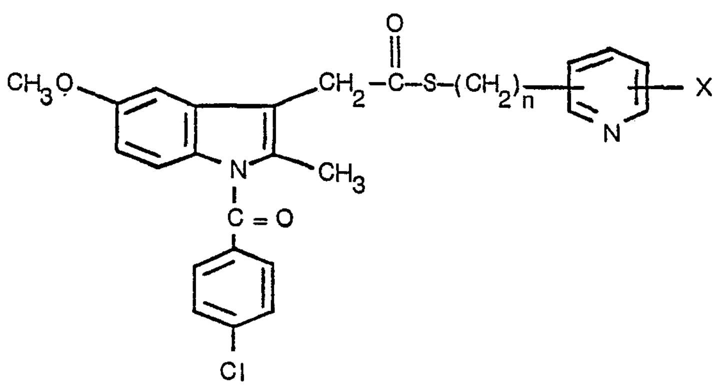

I

dans laquelle

X est H, F, Cl ou un groupe alkyle en $C_{1-3}$

n est nombre entier de 0 à 3,

ainsi que ses sels pharmacologiquement acceptables, procédé caractérisé en ce qu'il comprend:

a) la soumission de chlorure d'acide 1-(p-chlorobenzoyl)-5-méthoxy-2-méthyl-indol-3-acétique à une réaction avec un sel alcalin d'un composé de formule générale II

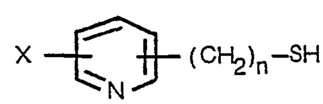

II

dans laquelle X et n ont la même signification que dans la formule I, dans un solvant organique inerte ou

b) la soumission d'un acide 1-(p-chlorobenzoyl)-5-méthoxy-2-méthyl-incol-3-acétique à une réaction avec un composé de la formule générale II ci-dessus en présence d'un carbodiimide, ou en présence de N,N-diméthyl-phosphoramidedichlorure en même temps qu'une base faible, dans un solvant organique inerte ou

c) la soumission d'un ester d'alkyle en $C_{1-4}$ d'acide 1-(p-chlorobenzoyl-5-méthoxy-2-méthyl-indol-3-acétique à une réaction avec un composé de la formule II ci-dessus en présence de triméthyl-aluminium dans un solvant organique inerte.